# EUROPEAN PATENT APPLICATION

(11) **EP 4 286 395 A1**
(43) Date of publication of application: **06.12.2023**
(21) Application number: 22745983.1
(22) Date of filing: 27.01.2022
(51) Int. Cl.: C07H 19/23

(54) **METHOD FOR PRODUCING IMIDAZOPYRIDINE DERIVATIVE**

(30) Priority: 29.01.2021 JP 2021013677
(71) Applicant: TAGCyx Biotechnologies Inc., Tokyo 1530041 (JP)
(72) Inventor: MUTO, Susumu, Tokyo 153-0041 (JP)
(74) Representative: Schön, Christoph
(86) International application number: PCT/JP2022/003130
(87) International publication number: WO 2022/163764

(57) **Abstract**

Provided is a method for producing an imidazopyridine derivative represented by the following Formula (I), wherein X is H, a substituted or unsubstituted propenyl group, a substituted or unsubstituted propynyl group, or a substituted or unsubstituted aryl group; and Y is H or OH, the method comprising a step of conducting a coupling reaction of a compound having a 5-X-2-thienyl group with 2-amino-3-nitro-4-chloropyridine under Suzuki-Miyaura coupling reaction conditions to obtain 2-amino-3-nitro-4-(5-X-2-thienyl)pyridine.

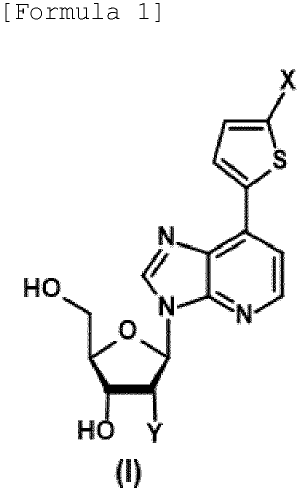

## Description

### [Technical Field]

The present invention relates to a method for producing an imidazopyridine derivative. Specifically, it relates to a method for producing the imidazopyridine derivative which includes a step of conducting a cross-coupling reaction without an organotin compound.

### [Background Art]

A cross-coupling reaction between an organometallic compound and an aryl halide using a transition metal catalyst is a highly versatile reaction that can convert a halogen moiety of the aryl halide to any of various carbon substituents using the aryl halide as a raw material which is a readily available electrophilic arylating agent, and is widely used also industrially as a method for introducing an aromatic substituent into an aromatic ring.

In this cross-coupling reaction, it is common to use mainly palladium (Pd) or nickel (Ni) as the transition metal catalyst. As the organometallic compound, on the other hand, a metal used (or a semimetal in some reactions) is very diverse and includes magnesium (Mg), zinc (Zn), tin (Sn), boron (B), and silicon (Si), and moreover it needs to be reacted in an amount used based on stoichiometry rather than a catalytic amount.

### [Citation List]

### [Non Patent Literature]

[NPL 1] Jana, Ranjan; Pathak, Tejas P.; Sigman, Matthew S., Advances in Transition Metal (Pd, Ni, Fe)-Catalyzed Cross-Coupling Reactions Using Alkyl-Organometallics as Reaction Partners, Chem. Rev., 2011, vol. 111(3), p. 1417-1492.
[NPL 2] Molnar, Arpad; Efficient, Selective, and Recyclable Palladium Catalysts in Carbon-Carbon Coupling Reactions, Chem. Rev., 2011, vol. 111(3), p. 2251-2320.

### [Summary of Invention]

### [Technical Problem]

When synthesizing a cross-coupling product on a large scale for commercialization oriented, zinc, tin, etc. discharged after the above cross-coupling reaction have problems that can lead to environmental pollution and a higher production cost during liquid waste treatment. In particular, organotin compounds are designated as "Specified Class I Designated Chemical Substances," which are recognized to have carcinogenicity, germ cell mutagenicity, and reproduction/development toxicity, in Japan under "Act on the Assessment of Releases of Specified Chemical Substances in the Environment and the Promotion of Management Improvements," and they are strictly regulated for their amounts to be used, methods in their use, and their emissions into the environment.

As described above, the cross-coupling reaction between the organometallic compound and the aryl halide using the transition metal catalyst is very versatile and useful. However, when a hazardous organometallic compound is used as the organometallic compound, conversion to another organometallic compound with lower toxicity and causing potentially less environmental pollution is an issue that should be considered in the future to ensure the safety of workers as well as to produce necessary industrial products in a sustainable manner while considering the global environment.

An unsymmetrical biaryl compound is present in a modified natural-type nucleobase and in a skeleton moiety of an artificial base. An artificial base pair formed of 7-(2-thienyl)-3H-imidazo[4,5-b]-pyridin-3-yl (hereinafter referred to as "Ds") and 4-(3-substituted-1-propynyl)-2-nitro-1H-pyrrol 1-1-yl (hereinafter referred to as "Px"), Ds-Px, has been developed as a highly lipid-soluble artificial base pair that enables PCR with high fidelity by Hirao et al. (JP 5424414 B2; Nucleic Acids Research, 2009(37), e14). Also, Hirao et al. have introduced single-stranded DNA containing only a few Ds as artificial bases into a library used in Selex, to conduct selection of aptamers utilizing the Ds-Px base pair. As a result, Hirao et al. have succeeded in obtaining the aptamers with extremely high affinity with Kd values of several pM with respect to several proteins (JP 6307675 B2; Michiko Kimoto, et al., Nature Biotechnology, 2013(31), 453-457).

Thus, Ds is the artificial base with high practicality and usefulness, but when synthesizing the skeleton of its base moiety, the cross-coupling reaction using the organotin compound (specifically, 2-tributylstannylthiophene) as the organometallic compound is employed (O 2006/077816). This may hinder continual and inexpensive mass synthesis of Ds at an industrial level.

Therefore, when synthesizing an imidazopyridine derivative that constitutes useful compounds such as Ds, it is useful for the environment and for ensuring the safety of technicians at work to replace the organotin compound used in the cross-coupling reaction with another organometallic compound that has less toxicity and contamination.

The present invention was made in view of these circumstances, and an objective of the present invention is to provide a method for producing the imidazopyridine derivative which includes a step of producing the unsymmetrical biaryl compound by the cross-coupling reaction not using the organotin compound.

### [Solution to Problem]

The first aspect of the present invention is a method for producing an imidazopyridine derivative represented by the following Formula (I), wherein the X is H, a substituted or unsubstituted propenyl group, a substituted or unsubstituted propynyl group, or a substituted or unsubstituted aryl group; and the Y is H or OH, the method comprising a step of conducting a coupling reaction of a compound having a 5-X-2-thienyl group with 2-amino-3-nitro-4-chloropyridine under Suzuki-Miyaura coupling reaction conditions to obtain 2-amino-3-nitro-4-(5-X-2-thienyl)pyridine.

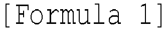

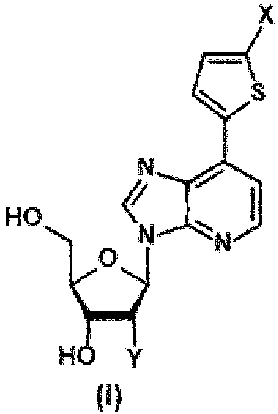

In one aspect of the above invention, preferably the X is H and the Y is H.

In one aspect of the above invention, preferably the X is H and the Y is OH.

### [Advantageous Effects of Invention]

According to the present invention, in the cross-coupling reaction for synthesizing an unsymmetrical biaryl compound which is also used as an intermediate for useful compounds such as Ds, there is no need to use an organotin compound which may cause problems for the safety of worker due to exposure and emissions after the reaction in a large scale synthesis, making a smaller load to the environment and synthesizing it more safely. This makes it possible to produce the imidazopyridine derivative, which is also used as the artificial bases such as Ds, more safely on the large scale.

### [Description of Embodiments]

With respect to a cross-coupling reaction between an organometallic compound and an aryl halide using a transition metal catalyst, there are known reaction examples with metals (semimetals) that are less hazardous and have less impact to environment under reaction conditions to obtain similar compounds. Also taking into consideration of ease of raw materials availability, studied have been reactions that are considered to be applicable. As a result, the present inventor has found in synthesizing a desired imidazopyridine derivative that the Suzuki-Miyaura coupling reaction (Norio Miyaura, Akira Suzuki, Chem. Rev., 1995, vol. 95, p. 2457-2483) can be applicable as a coupling reaction, and has arrived at completion of the present invention.

In the Suzuki-Miyaura coupling reaction, boron, a semimetal, is used as a metal in the organometallic compound. Boron is considered to be useful as a raw material used for sustainable mass synthesis because of its low toxicity and low impact to the environment. An organoboron compound used in the Suzuki-Miyaura coupling reaction is stable in water and in air, easy to handle, and has also an advantage that the reaction proceeds even in solvent systems containing water.

A general formula of the coupling reaction of a compound having a 5-X-2-thienyl group with 2-amino-3-nitro-4 chloropyridine in the present embodiment is shown in (Formula 2) below. The starting material, the 2-amino-3-nitro-4-chloropyridine, is a known compound, and can be synthesized by using a known method (e.g., Rec. Trav. Chim., 1963, vol. 88, p. 1263-1274). 2-Thiopheneboronic acid is sold by several domestic and foreign reagent manufacturers and is readily available. In the cross-coupling reaction of the present embodiment, boronic ester of thiophene can also be used in place of thiopheneboronic acid. 2-Bromo-5-X-thiophene can be synthesized from 2-bromothiophene by a known method. Position-2 boronic acid ester of the thiophene having a substituent X can be synthesized from the 2-bromo-5-X-thiophene by a known method.

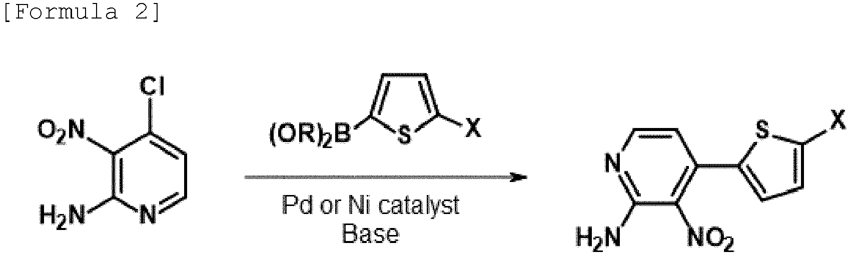

In the above scheme, X can be selected appropriately. In the present embodiment, it can be H, a substituted or unsubstituted propenyl group, a substituted or unsubstituted propynyl group, or a substituted or unsubstituted aryl group. In a preferred aspect, X in the above formula is H. In the above scheme, (OR)₂ is (OH)₂, or a cyclic or acyclic ester formed from a diol.

As a catalyst for the above cross-coupling reaction applicable to the present embodiment, zero- or II-valent complexes of Pd or II-valent complexes of Ni can also be used. As the zero-valent complexes of Pd, Pd(PPh₃)₄, Pd₂(dba)₃, Pd/C, etc. are generally used, and as the II-valent complexes of Pd, PdCl₂(dppf), PdCl₂(Py₃)₂, PdCl₂[P(o-tolyl)₃]₂, PdCl₂(PPh₃)₂, Pd(OAc)₂, etc. can be used.

A base to be added to the above cross-coupling reaction applicable to the present embodiment includes Na₂CO₃, K₂CO₃, Cs₂CO₃, Ba(OH)₂, K₃PO₄, TlOH, KF, CsF, Bu₄NF, NaOH, KOH, NaOMe, TEA (triethylamine), DIEA (diisopropylethylamine), etc.

A solvent used in the above cross-coupling reaction applicable to the present embodiment includes benzene, toluene, DME, THF, dioxane, DMF, water (as a mixed solvent with an organic solvent), acetonitrile, alcohol (MeOH, EtOH, i-PrOH, BuOH), etc.

Since an organotin compound is not used in the present embodiment, it can bring about a greater effect of reducing an amount of liquid waste containing the organotin compound, particularly when conducting the cross-coupling reaction on a large scale. In the present description, "synthesis on the large scale" refers to as the synthesis on a reaction scale that is difficult to work on in a laboratory, and that is difficult to use local exhaust ventilation facilities and the like that are generally used in the laboratory, more specifically, on the reaction scale where volume of the reaction vessel is 10 to 20 L or more.

The desired unsymmetrical biaryl compound can be synthesized by the present embodiment. The unsymmetrical biaryl compound obtained can be applied, for example, to the synthesis of an artificial nucleoside. For example, 7-(2-thienyl)-3-(2-deoxy-1-β-D-ribofuranosyl)-3H-imidazo[4,5-b]pyridine, which is a deoxyribonucleoside having the artificial base Ds as the base, can be synthesized from 2-amino-3-nitro-4-(2-thienyl)pyridine obtained by using the cross-coupling reaction described above by a known method (e.g., WO 2006/077816). Similarly, ribonucleoside having the artificial base Ds as the base can be synthesized by using a known reaction.

Hereinafter, one embodiment of the present invention will be specifically described with reference to Examples. These are not intended to limit technical scope of the present invention, and one skilled in the art can appropriately modify various conditions based on the description herein, which are included in the technical scope of the present invention. In particular, one skilled in the art can easily understand that an equivalent amount of each substance and a reaction concentration can be optimized as appropriate depending on the reaction scale.

### [Examples]

### [Example 1] Synthesis of thiophene-2-ylboronic acid pinacol

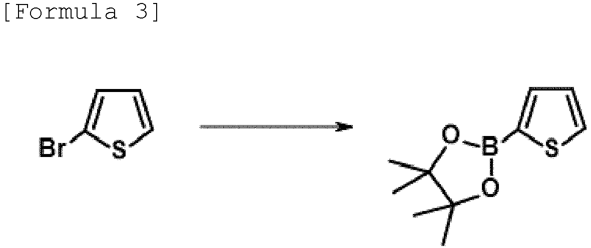

The 2-bromothiophene (80 mmol) was dissolved in 1,4-dioxane (200 mL), and under an argon atmosphere, bis(pinacolato)diboron (40.5 g, 160 mmol), [1,1'-bis(diphenylphosphino)ferrocene]palladium(II)dichloride dichloromethane adduct (4.89 g, 5.98 mmol), and potassium acetate (23.5 g, 239 mmol) were added, and stirred at 90°C for 2 hours. After confirming disappearance of the raw materials, a mixture solution of water and heptane/ethyl acetate = 1/1 solution was added to a reaction solution, stirred, and then separated. After an organic layer was washed with brine, dried with anhydrous sodium sulfate, and filtered, filtrate was concentrated under reduced pressure. The residue was purified by silica gel chromatography to obtain the thiophene-2-ylboronic acid pinacol (8.0 g to 10.0 g).

### [Example 2] Synthesis of 2-amino-3-nitro-4-(2-thienyl)pyridine using Suzuki-Miyaura coupling reaction

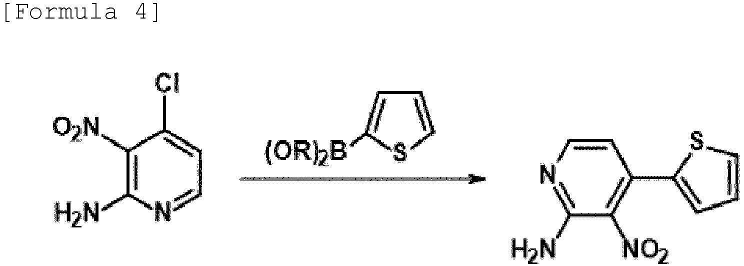

The thiophene-2-ylboronic acid pinacol synthesized in Example 1 (in the above scheme, (OR)₂ is -O-C(CH₃)₂C(CH₃)₂-O-) or the 2-thiopheneboronic acid (in the above scheme, OR is OH)(41 mmol), and the 2-amino-3-nitro-4-chloropyridine (5.7 g, 33 mmol) are solved in 1,4-dioxane/water = 10/1 (242 mL), then degassed, and under the argon atmosphere, cesium carbonate (21 g, 66 mmol) and tetrakis(triphenylphosphine)palladium(O) (1.9 g, 1.6 mmol) are added and stirred at 90°C for 1 hour to 2 hours. After confirming the disappearance of the raw material, the mixture solution of water and ethyl acetate is added to a reaction solution, stirred, and then separated. The aqueous layer is extracted once with ethyl acetate, the combined organic layer is dried with the anhydrous sodium sulfate, filtered, and the filtrate is concentrated under the reduced pressure. The concentrated residue is purified by the silica gel chromatography to obtain the 2-amino-3-nitro-4-(2-thienyl)pyridine (5.1 g to 6.5 g, 70% to 90% yield).

### [Example 3] Synthesis of 7-(2-thienyl)-3-(2-deoxy-1-β-D-ribofuranosyl)-3H-imidazo[4,5-b]pyridine (Ds)

With the 2-amino-3-nitro-4-(2-thienyl)pyridine produced in Example 2 as the raw material, 7-(2-thienyl)-3-(2-deoxy-1-β-D-ribofuranosyl)-3H-imidazo[4,5-b]pyridine (Ds) can be obtained under known conditions (e.g., the method described in WO 2006/077816).

## Claims

1. A method for producing an imidazopyridine derivative represented by the following Formula (I): 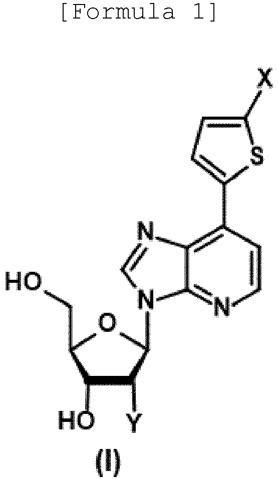
wherein the X is H, a substituted or unsubstituted propenyl group, a substituted or unsubstituted propynyl group, or a substituted or unsubstituted aryl group; and
the Y is H or OH,
the method comprising a step of conducting a coupling reaction of a compound having a 5-X-2-thienyl group with 2-amino-3-nitro-4-chloropyridine under Suzuki-Miyaura coupling reaction conditions to obtain 2-amino-3-nitro-4-(5-X-2-thienyl)pyridine.

2. The method according to claim 1, wherein the X is H and the Y is H.

3. The method according to claim 1, wherein the X is H and Y is OH.
